# EUROPÄISCHE PATENTSCHRIFT

(11) **EP 1 279 664 B1**
(45) Veröffentlichungstag und Bekanntmachung des Hinweises auf die Patenterteilung: **05.10.2005**
(21) Anmeldenummer: 02012777.5
(22) Anmeldetag: 10.06.2002
(51) Int. Cl.: C07C 303/32, C07C 309/20

(54) **Verfahren zur Herstellung von Salzen der Methallylsulfonsäure**
Process for the preparation of methallylsulphonic acid salts
Procédé pour la préparation de sels de l'acide méthallylsulfonique

(30) Priorität: 24.07.2001 DE 10135907
(43) Veröffentlichungstag der Anmeldung: 29.01.2003
(73) Patentinhaber: Oxeno Olefinchemie GmbH, 45772 Marl (DE)
(72) Erfinder: Esser, Peter Ernst, Dr., 85609 Aschheim (DE); Steinbeisser, Hartmut, Dr., 45772 Marl (DE); Kerker, Lothar, Dr., 48249 Dülmen (DE); Kuppinger, Franz-Felix, Dr., 45768 Marl (DE); Werninger, Claus York, Prof. Dr., 25524 Itzehoe (DE); Büschken, Wilfried, Dr., 45721 Haltern (DE)

(56) Entgegenhaltungen:
- EP-A- 0 325 975
- EP-A- 0 531 865
- DE-A- 1 804 833
- DE-A- 1 965 002
- DE-A- 2 530 711

## Beschreibung

Gegenstand der vorliegenden Erfindung ist ein Verfahren zur Herstellung von Methallylsulfonsäuresalzen durch Sulfonierung von Isobuten mit Lewis-Base/Schwefeltrioxid-Komplexen und anschließende Neutralisation.

Salze der Methallylsulfonsäure werden als Comonomere bei der Synthese von Faservorprodukten eingesetzt, insbesondere zur Modifizierung von Polyacrylnitrilen. Darüber hinaus finden diese Salze Anwendung bei der Herstellung von Dispersionen.

Technisch werden Salze der Methallylsulfonsäure, ausgehend von Isobuten, auf zwei verschiedenen Wegen hergestellt:

Beim ersten Syntheseweg wird Isobuten mit Chlor zum Zwischenprodukt Methallylchlorid umgesetzt. Diese Verbindung reagiert mit Sulfiten zu den Zielprodukten. Beispielsweise liefert die Umsetzung von Methallylchlorid mit Natriumsulfit Natriummethallylsulfonat und Natriumchlorid. Nachteilig bei dieser Synthese ist, dass die molare Ausbeute an Zielprodukt nur 85 %, bezogen auf Isobuten beträgt und anorganische Chloride als Koppelprodukte und organische Chlorverbindungen als Nebenprodukte anfallen.

Bei dem anderen Syntheseweg wird Schwefeltrioxid an Isobuten addiert und anschließend die dabei entstehende Methallylsulfonsäure mit einer Base zum entsprechenden Salz umgesetzt. Aus der Literatur sind viele verschiedene Verfahren für diese Reaktionsfolge bekannt. Wegen der höheren Selektivität für die Bildung der Methallylsulfonsäure wird die Sulfonierung von Isobuten nicht mit Schwefeltrioxid, sondern mit einem Komplex aus Schwefeltrioxid und einer Lewis-Base durchgeführt.

Technische Prozesse für die Herstellung von Salzen der Methallylsulfonsäure, in der Regel das Natriumsalz, umfassen folgende Stufen:
a) Herstellung einer Lösung von einem Komplex, bestehend aus Schwefeltrioxid und einer Lewis-Base und gegebenenfalls eines Lösungsmittels
b) Sulfonierung von Isobuten mit der in der Stufe a) hergestellten Lösung
c) Umsetzung der in Stufe b) entstandenen Säuren zu ihren Salzen
d) Abtrennung und Reinigung des Methallylsulfonats
e) Zurückgewinnung und Reinigung des Komplexbildners und gegebenenfalls eines Lösungsmittel.

Die in der Technik angewendeten Verfahren lassen sich in zwei Gruppen einteilen. Die erste Gruppe hat gemeinsam, dass die Herstellung des SO₃/Lewis-Säure-Komplexes und dessen Umsetzung mit Isobuten in einem zusätzlichen Lösungsmittel durchgeführt wird. Übliche Lösungsmittel sind Ethylenglycoldimethylester und halogenierte Kohlenwasserstoffe wie Methylenchlorid, Chloroform und 1,2-Dichlorethan. Nachteilig bei diesen Verfahren ist, dass neben der Lewis-Säure auch ein Lösungsmittel abgetrennt und vor Rückführung in den Prozess gereinigt werden muss. Weiterhin entstehen Verluste an Lösungsmittel durch chemische Umsetzungen. Beispielsweise werden halogenierte Kohlenwasserstoffe bei der Neutralisation in geringem Maße hydrolysiert. Dadurch entstehen organische Nebenprodukte, die abgetrennt werden müssen, und ein wässriger Abwasserstrom, der Chloride enthält. Diese Stoffe bedingen zusätzliche Entsorgungskosten.

Bei der zweiten Verfahrensgruppe wird kein zusätzliches Lösungsmittel eingesetzt. Die flüssige Lewis-Säure wird im Überschuss eingesetzt und dient nicht nur als Komplexbildner, sondern auch als Lösungsmittel bzw. Suspensionsmittel für den Schwefeltrioxid/Lewis-Säure-Komplex.

In DE 1 804 833 wird ein Verfahren zur Herstellung von Salzen der Methallylsulfonsäure ohne Verwendung eines zusätzlichen Lösemittel dargelegt. Dabei wird Isobuten mit einem Komplex aus Schwefeltrioxid und einem N,N-dialkylsubstituierten Amid einer aliphatischen Carbonsäure oder einem N-alkylierten Lactam in einem Überschuss des Komplexbildners als Reaktionsmedium in einem Verhältnis von mindestens einem Mol Isobuten zu einem Mol Schwefeltrioxid, bei Temperaturen zwischen -20 bis +60 °C, umgesetzt. Das erhaltene Reaktionsgemisch wird mit einer wässrigen Base neutralisiert. Der Reaktionsschritt der Neutralisation ist so durchzuführen, dass keine Nebenreaktionen auftreten sollen. Einziger Hinweis auf eine solche Reaktionsführung ist die Verwendung von verdünnten Laugen oder schwachen Alkalien wie Soda zur Neutralisation der Reaktionsmischung. Aus der Wasserphase wird nach vollständiger oder teilweiser Eindampfung das Zielprodukt abgetrennt. Die Ausbeute an gelblichen Rohprodukt, bezogen auf Schwefeltrioxid, beträgt bis zu 97 %. Die Ausbeute an einem reinen, weißen Produkt ist mindestens 80 %. Die Bildung von Nebenprodukten kann durch die Sulfonierungsreaktion oder die Neutralisation erfolgen; so ist insbesondere die Hydrolyse des Komplexbildners als Nebenreaktion zu nennen, die das Produkt verunreinigt und gleichzeitig die Wirtschaftlichkeit des Verfahrens durch den Stoffverlust in Frage stellt.

Es bestand somit die Aufgabe, ein wirtschaftliches Verfahren zur Herstellung von Methallylsulfonsäuresalzen zu entwickeln, bei dem die Bildung von Nebenprodukten vermindert, bzw. die Ausbeute an Zielprodukt erhöht wird.

Es wurde nun überraschend gefunden, dass der Verlust an Komplexbildner, der Laugenverbrauch und der Aufwand für die Abtrennung des Zielproduktes verringert werden kann, wenn das aus der Sulfonierung von Isobuten mit einem Schwefeltrioxid-Lewis-Basen-Komplex entstandene Reaktionsgemisch mit Wasser vor der Neutralisation des Gemisches verdünnt wird.

Gegenstand der vorliegenden Erfindung ist daher ein Verfahren zur Herstellung von Salzen der Methallylsulfonsäure durch Umsetzung von Isobuten mit einem Schwefeltrioxid/Lewis-Base-Komplex in einem organischen Lösungsmittel, Neutralisation des entstehenden Reaktionsgemisches mit einer Base und Abtrennung des Methallylsulfonsäuresalz, wobei das
a) Isobuten mit einem Schwefeltrioxid/Lewis-Base-Komplex bei - 20 bis 80 °C und 1 bis 15 bar sulfoniert,
b) das Reaktionsgemisch mit Wasser verdünnt,
c) das verdünnte Reaktionsgemisch mit einer Base auf einen pH von 6-8 neutralisiert und
d) das Methallylsulfonsäuresalz aus dem neutralisierten Reaktionsgemisch gewonnen wird, wobei das organische Lösungsmittel mit der als Komplexbildner eingesetzten Lewis-Base identisch ist.

Die Sulfonierung wird in einem organischen Lösungsmittel wie z. B. einer Lewis-Base durchgeführt. Die als Komplexbildner und als Lösungsmittel eingesetzten Lewis-Basen sind identisch.

Im erfindungsgemäßen Verfahren werden bevorzugt als Komplexbildner für Schwefeltrioxid und als Lösemittel N,N-dialkylierte Carbonsäureamide, wie beispielsweise N,N-Diethylacetamid, N,N-Dimethylacetamid und N,N-Dimetylformamid (DMF), N-alkylierte Lactame, wie beispielsweise N-Methylpyrrolidon, Dialkylsulfoxide oder cyclische Sulfoxide, beispielsweise Dimethylsulfoxid, cylische Sulfone oder Dialkylsulfone, cyclische Ether, wie beispielsweise Dioxan, verwendet. Eine bevorzugte Lewis-Base als Komplexbildner und Lösemittel ist N,N-Dimethylformamid (DMF).

Zur Komplexierung von Schwefeltrioxid bzw. als Lösungsmittel kann auch ein Gemisch aus zwei oder mehreren der obengenannten Lewis-Basen eingesetzt werden. Es ist jedoch wegen der leichteren Rückgewinnung der Hilfsstoffe zweckmäßig, nur eine Lewis-Base zu verwenden.

Für die Herstellung des Schwefeltrioxid-Komplexes können flüssiges Schwefeltrioxid, gasförmiges Schwefeltrioxid, das beispielsweise aus Oleum herausdestilliert wird, oder andere Gasgemische, die Schwefeltrioxid enthalten, eingesetzt werden.

Für die Herstellung des Komplexes können Gasgemische verwendet werden, die neben Schwefeltrioxid andere Gase enthalten, die unter den Reaktionsbedingungen der Komplexierung nicht mit dem Komplexbildner reagieren bzw., wenn überhaupt, mit ihm nur Komplexe bilden, die deutlich schwächer als die entsprechenden Schwefeltrioxid-Komplexe sind.

Solche Gasanteile können beispielsweise Stickstoff, Sauerstoff, Kohlendioxid oder Schwefeldioxid sein. Ein bevorzugtes Einsatzgas ist das Kontaktofengas einer Schwefelsäureanlage, das beispielsweise folgende Zusammensetzung aufweist: 7,5 Vol-% SO₃; 0,2 Vol-% SO₂; 13,0 Vol-% O₂; 79,3 Vol-% N₂. Es sei jedoch betont, dass das Kontaktgas auch ganz andere Zusammensetzungen besitzen kann. Für die Herstellung von SO₃-Komplexen im erfindungsgemäßen Verfahren werden insbesondere Kontaktofengase mit relativ hohen SO₃-Gehalt und niedrigem SO₂-Gehalt bevorzugt. Dies hat den Vorteil, dass wegen der geringeren Abgasmenge weniger Komplexbildner ausgetragen wird und die Bildung des SO₂-Komplexes gering ist. Die Bildung des SO₂-Komplexes ist unerwünscht, da er bei der Neutralisation einen höheren Laugenverbrauch bedingt und die entstandenen Sulfite vom Zielprodukt abgetrennt werden müssen.

Um eine homogene Lösung des Schwefeltrioxid/Lewis-Base-Komplexes in der Lewis-Base zu erhalten, muss die Lewis-Base im Überschuss eingesetzt werden. Bei der Verwendung von DMF als Komplexbildner und Lösungsmittel liegt das molare Verhältnis zwischen DMF und SO₃ im Bereich von 100/1 bis 5/1, insbesondere im Bereich von 20/1 bis 6/1.

Schwefeltrioxid bildet mit Wasser Schwefelsäure, die sich unter Sulfonierungsbedingungen mit Isobuten nicht zur Methallylsulfonsäure umsetzt, jedoch Nebenreaktionen bewirken kann. Bei der Neutralisation entstehen aus ihr Sulfate, die wie die anderen Nebenprodukte abgetrennt werden müssen. Um eine hohe Ausbeute zu erhalten ist es daher erforderlich, die Lewis-Base möglichst wasserfrei einzusetzen, z. B. durch Trocknung über Molsiebe. Dies gilt auch für aus dem Prozess abgetrennte und nach Aufarbeitung wieder zugeführte Lewis-Base. Weiterhin ist darauf zu achten, dass die Lewis-Base keine anderen protischen Verbindungen, wie beispielsweise Alkohole, oder andere Stoffe, die mit Schwefeltrioxid reagieren könnten, als Verunreinigung enthält, weil dadurch die Ausbeute gemindert und der Aufarbeitungsaufwand erhöht wird.

Die Komplexbildung kann diskontinuierlich oder kontinuierlich in einem Rührkessel, einer Rührkesselkaskade, in einer oder mehreren Blasensäulenreaktoren durchgeführt werden. Bei Einsatz von flüssigen Schwefeltrioxid wird die Komplexierung in einem oder mehreren hintereinandergeschalteten Rührkessel durchgeführt. Die Verweilzeiten betragen dabei 1 bis 3 h.

Wird ein Schwefeltrioxid-Gasgemisch mit einem hohen Inertgasanteil eingesetzt, wie beispielsweise ein Kontaktofengas einer Schwefelsäure-Anlage, wird die Komplexierung vorzugsweise in einer oder mehreren hintereinandergeschalteten Blasensäule(n) durchgeführt. Die Reaktion kann bei Überdruck oder Normaldruck ablaufen. Um eine möglichst vollständige Absorption des Schwefeltrioxids zu erreichen und die Verluste an Lewis-Base zu minimieren, wird die Gaseinleitungsgeschwindigkeit klein gehalten. Die Reaktionszeit beträgt 1 bis 10 h, vorzugsweise 2 bis 8 h. Die Reaktionstemperaturen liegen im Bereich von 5 bis 50 °C, insbesondere im Bereich von 8 bis 27 °C, ganz besonders im Bereich von 10 bis 15 °C.

Die Sulfonierung kann mit einem Isobutenüberschuss bezüglich des eingesetzten SO₃ von 5 bis 60 Mol-%, insbesondere 5 bis 20 % erfolgen. Zunächst wird bei Temperaturen zwischen -20 und 25 °C, insbesondere 5,0 und 10 °C Isobuten flüssig oder gasförmig zum Schwefeltrioxid/Lewis-Base-Komplex, optional in einem der o. g. Lösungsmittel gegeben. Anschließend wird aufgeheizt und die Umsetzung im Temperaturbereich bis zu 80 °C, bevorzugt 45 bis 55 °C zu Ende geführt. Um das Isobuten in Lösung zu halten, wird die Reaktion unter Isobuteneigendruck durchgeführt.

Das Mischen der Reaktionskomponenten bzw. das erfindungsgemäße Verfahren selbst können diskontinuierlich oder kontinuierlich durchgeführt werden.

Bei einem diskontinuierlichen Verfahren in einem Rührkessel wird beispielsweise in eine Schwefeltrioxid-DMF-Lösung bei einer Temperatur zwischen -20 und 25 °C, insbesondere 5 und 10 °C und ganz besonders bei 7 bis 9 °C, innerhalb von 0,2 bis 1,0 h, insbesondere 0,4 bis 0,6 h, Isobuten gasförmig oder bevorzugt flüssig eindosiert. Innerhalb von 0,05 bis 1,0 h, insbesondere 0,15 bis 0,30 h, wird der Reaktorinhalt auf 43 bis 48 °C, insbesondere 45 bis 46 °C, gebracht. Nach einer weiteren Reaktionszeit von 0,05 bis 0,3 h bei dieser Temperatur wird eine Endtemperatur von 49 bis 52 °C eingestellt und diese 0,9 bis 1,5 h, insbesondere 1,0 bis 1,3 h lang konstant gehalten.

Ein kontinuierliches Verfahren könnte beispielsweise in folgender Weise durchgeführt werden: In eine Schwefeltrioxid-DMF-Lösung wird unter Druck im Temperaturintervall von 5 bis 10 °C Isobuten mit Hilfe eines Statikmischers eingemischt. Das Reaktionsgemisch wird in einer Rohrschlange auf 30 °C gebracht und anschließend in zwei hintereinandergeschalteten Rührreaktoren umgesetzt, von denen der erste bei 45 °C und der zweite bei 50 °C betrieben wird. Eine andere Möglichkeit bestünde darin, das Reaktionsgemisch in einer dreistufigen Rührkesselkaskade mit einer Verweilzeit von je einer halben Stunde umzusetzen, wobei der erste Reaktor bei einer Temperatur von 45 °C und die beiden anderen bei 50 °C betrieben würden.

Bei beiden Varianten wird nach Beendigung der Sulfonierung auf Normaldruck entspannt. Optional wird ein leichtes Vakuum angelegt. Aus dem Entspannungsgas kann ein Teil des im Überschuss eingesetzten Isobuten zurückgewonnen und in den Prozess zurückgeführt werden.

Optional kann die Druckentspannung und die Zurückgewinnung des überschüssigen Isobutens nach der Wasserzugabe oder nach der Neutralisation erfolgen.

Eine weitere Möglichkeit zur Abtrennung des nicht umgesetzten Isobutens besteht darin, dass unter Isobuteneigendruck entweder nach Verdünnen des Sulfonierungsgemisches mit Wasser oder nach Verdünnen des Sulfonierungsgemisch mit Wasser und nach anschließender Neutralisation das Isobuten als zweite entstandene flüssige Phase abgezogen wird.

Im erfindungsgemäßen Verfahren wird das obige Reaktionsgemisch, in dem die Methallylsulfonsäure enthalten ist, vor der Neutralisation mit einer Lauge mit Wasser verdünnt. Dies kann diskontinuierlich oder kontinuierlich durchgeführt werden. Dabei kann Wasser unter Vermischen zum Reaktionsaustrag der Sulfonierung zudosiert werden. Es ist jedoch auch möglich umgekehrt zu verfahren, d. h., das Reaktionsgemisch der Sulfonierung ins Wasser zu geben. Weiterhin ist es möglich, den Reaktionsaustrag der Sulfonierung mit Wasser direkt im vorgesehenen Verhältnis zu mischen.

Wasser wird beispielsweise während 0,1 bis 1,0 h, insbesondere während 0,2 bis 0,5 h zudosiert. Die Temperatur des Gemisches wird dabei zwischen 20 und 80 °C, insbesondere zwischen 30 bis 55 °C gehalten. Eine zusätzliche Mischzeit ist nicht erforderlich. Die zugesetzte Wassermenge ist abhängig von der zur Sulfonierung eingesetzten Schwefeltrioxidmenge. Je 1 kg Schwefeltrioxid wird mit 2 bis 10 kg Wasser, insbesondere mit 3 bis 5 kg Wasser, entsprechend den Massenverhältnissen 1:2 bis 1:10 bzw. 1:3 bis 1:5, verdünnt.

Die Neutralisation der saueren Stoffe, wie Sulfonsäuren und Schwefelsäure, geschieht durch Zugabe von anorganischen oder organischen Basen wie der basisch wirkenden Verbindungen der Alkali- und Erdalkalimetalle. Diese werden in Form ihrer Carbonate, Hydrogencarbonate oder Hydroxide eingesetzt. Das Neutralisationsmittel kann in fester Form oder bevorzugt als Lösung, insbesondere als wässrige eingesetzt werden. Die wässrigen Lösungen können 5 bis 40 %-ig sein. Insbesondere wird Natronlauge im Konzentrationsbereich von 1 bis 30 %, ganz besonders von 20 bis 30% verwendet. Das Neutralisationsmittel wird in einer Menge zugesetzt, dass die resultierende Mischung einen pH-Wert von 6 bis 8, insbesondere von 6,5 bis 7,5 besitzt. Im günstigsten Fall ist je Mol Schwefeltrioxid ein Mol einer einwertigen Base notwendig. Da sich jedoch ein Teil des Schwefeltrioxids zu Schwefelsäure umsetzen könnte und gegebenenfalls außer Sulfonsäure auch andere sauere Stoffe vorliegen, wie beispielsweise Schwefeldioxid, ist die benötigte Laugenmenge meistens größer.

Das Kation der Base stellt auch das Kation des erhaltenen Methallylsulfonsäuresalzes dar.

Sind Methallylsulfonsäuresalze mit anderen Kationen erwünscht, können diese durch Ionenaustausch hergestellt werden. Beispielsweise kann ein Kationenaustausch mit Hilfe eines Kationenaustauscher durchgeführt werden.

Die Neutralisation erfolgt pH-geregelt bei einer Temperatur zwischen 20 und 80 °C, insbesondere zwischen 40 und 60 °C. Die Neutralisationszeit beträgt 0,1 bis 1,0 h, insbesondere 0,2 bis 0,7 h.

Das flüssige neutrale Reaktionsgemisch ist in der Regel homogen und besteht im Wesentlichen aus Wasser, Lewis-Base, Methallylsulfonat, anderen Sulfonaten und gegebenenfalls aus Sulfiten und Zersetzungsprodukten der Lewis-Base.

Aus dieser Lösung kann das Zielprodukt, das gewünschte Salz der Methallylsulfonsäure nach verschiedenen Weisen abgetrennt werden:
a) Das neutralisierte Reaktionsgemisch wird destillativ aufkonzentriert, d. h. eingedampft und aus dem Rückstand, gegebenenfalls nach Wasserwäsche, wird das reine Zielprodukt durch Umkristallisation aus einem Lösungsmittel oder Lösungsmittelgemisch, das nicht die zur Reaktion eingesetzte Lewis-Base enthalten muss, gewonnen.
b) Das neutralisierte Reaktionsgemisch wird destillativ so aufkonzentriert, das durch die Verdampfung das Zielprodukt auskristallisiert. Gegebenenfalls wird das abgetrennte Produkt gewaschen und/oder umkristallisiert.
c) Das neutralisierte Reaktionsgemisch gekühlt und das entstandene Kristallisat abgetrennt.
d) Das neutralisierte Reaktionsgemisch wird durch Extraktion von der Lewis-Base abgetrennt. Aus der verbleibenden wässrigen Phase wird das Zielprodukt nach teilweiser Eindampfung durch Kristallisation gewonnen.
e) Aus dem neutralisierten Reaktionsgemisch wird das Zielprodukt als wässrige Lösung durch Elektrodialyse abgetrennt. Aus der wässrigen Lösung erfolgt die Isolierung des Zielproduktes durch Kristallisation nach destillativer Aufkonzentrierung.

Im erfindungsgemäßen Verfahren wird, wenn Natriummethallylsulfonat das Zielprodukt ist und DMF als Lewis-Base eingesetzt wird, das Produkt bevorzugt durch Kühlkristallisation nach Abtrennung des Wassers und eines Teils des DMF gewonnen.

Die Aufkonzentration durch Destillation erfolgt bevorzugt im Temperaturbereich von 110 °C bis 140 °C, insbesondere im Bereich 110 °C bis 120 °C. Es werden 50 bis 70 %, insbesondere 50 bis 60 %, ganz besonders 50 bis 56 % des Reaktionsgemisches abdestilliert. Das Destillat ein Wasser-DMF-Gemisch, besteht vorwiegend aus Wasser.

Anschließend wird die ggf. aufkonzentrierte Reaktionsmischung abgekühlt, beispielsweise in einem Kristallisator mit Rührer. Bei circa 110 °C beginnt die Kristallisation. Zur Erhöhung der Kristallisationsausbeute wird die Lösung auf 20 bis 30 °C abgekühlt. Um ein reines und gut abfiltrierbares Kristallisat zu erhalten, ist es zweckmäßig, die Lösung langsam abzukühlen. Im Temperaturbereich von 110 °C bis 80 °C ist eine Abkühlungsrate von 5 bis 20 °C/h, insbesondere eine von 5 bis 10 °C/h vorteilhaft. Im Temperaturbereich von 80 °C bis zur Endtemperatur beträgt die Abkühlungsrate 20-50 °C/h, insbesondere 25 bis 35 °C/h.

Optional kann die Abtrennung des Methallylsulfonsäuresalzes, z. B. Natriummethallylsulfonat aus einer wässrigen DMF-Lösung durch Elektrodialyse im Temperaturbereich 20 °C bis 100 °C, insbesondere im Bereich von 20 °C bis 60 °C, unter Verwendung von Ionenaustauschermembranen erfolgen. Man erhält eine Lösung (Konzentrat), die hauptsächlich aus dem gewünschten Salz (z. B. Natriummethallylsulfonat) und Wasser besteht, und eine Lösung (Retentat), die hauptsächlich aus DMF und Wasser besteht. Während der Elektrodialyse erhöht sich die Konzentration des Zielproduktes. Um ein Auskristallisation des Zielproduktes und eine Belegung oder Verstopfung der Membrane zu verhindern, kann es zweckmäßig sein, die Temperatur im obengenannten Bereich zu erhöhen.

Wenn die wässrige Methallylsulfonat-Lösung nicht als solche verwendet wird, kann aus ihr gegebenenfalls nach weiterer destillativer Aufkonzentrierung das Zielprodukt durch Kristallisation abgetrennt werden. Die Kristallisation erfolgt unter Einhaltung der oben genannten Abkühlungsraten.

Das Kristallisat wird von der Mutterlauge durch Filtration oder durch Zentrifugieren abgetrennt. Das Kristallisat kann mit einem Lösemittel, insbesondere Wasser gewaschen werden. Wird mit Wasser gewaschen, beträgt die zum Waschen verwendete Wassermenge 5 bis 30 %, bevorzugt 10 bis 20 % der Kristallmasse. Das Kristallisat besteht nach Trocknung zu über 99 %, bevorzugt zu über 99,5 % aus dem gewünschten Methallylsulfonat.

Die Mutterlauge und die Waschlösung können getrennt oder gemeinsam aufkonzentriert und in den Kristallisator zurückgeführt werden. Es ist jedoch auch möglich, diese Ströme zusammen mit dem neutralisierten Sulfonierungsaustrag aufzukonzentrieren.

Ein Teil der Mutterlauge und/oder des Waschwassers wird zur Abtrennung der Nebenprodukte ausgeschleust. Dies geschieht, insbesondere bei der Mutterlauge, zweckmäßig nach der Aufkonzentrierung.

Die bei der Aufkonzentration des neutralisierten Reaktionsgemisches anfallende Lewis-Base, d. h. die vereinten Brüden oder das Retentat der Elektodialyse, die hauptsächlich aus Wasser und Lewis-Base bestehen, können destillativ in Wasser, Lewis-Base und Nebenprodukten getrennt werden. Nebenprodukte sind beispielsweise Dimethylamin, entstanden durch Hydrolyse von Lewis-Base.

Die zurückgewonnene Lewis-Base kann nach Entfernung von Wasserspuren, beispielsweise durch Trocknung über Molsiebe, in den Prozess zurückgeführt werden. Das bei der destillativen Auftrennung erhaltene Wasser kann zur Verdünnung des Sulfonierungsgemisch, zur Herstellung der wässrigen Lauge und/oder als Waschflüssigkeit genutzt werden.

Das hergestellte Salz, insbesondere Natriummethallylsulfonat, kann nach Trocknung als rieselfähiges Kristallisat oder nach Auflösung in Wasser als wässrige Lösung verwendet werden. Die Trocknung erfolgt nach bekannten Verfahren.
Die nach dem erfindungsgemäßen Verfahren hergestellten Salze der Methallylsulfonsäure, insbesondere das Natriumsalz, finden Verwendung als Comonomere bei der Herstellung von Polymerdispersionen und Faservorprodukten, wie beispielsweise modifiziertes Polyacrylnitril. Durch den Einbau des Methallylsulfonats in das Polymere wird dessen Anfärbbarkeit verbessert.

Das erfindungsgemäße Verfahren weist folgende Vorteile auf:
Es geht nur eine geringe Menge an Komplexbildner verloren, was die Stoffkosten gering hält. Die geringen Mengen an Nebenprodukten erleichtern die kostengünstige Abtrennung des Zielproduktes und die Rückgewinnung des Komplexbildners in reiner Form. Weiterhin ist es möglich, ein preiswertes Kontaktofengas einer Schwefelsäureanlage als Schwefeltrioxidquelle für die Herstellung des Schwefeltrioxid/Lewis-Base-Komplexes zu nutzen.

Die folgenden Beispiele sollen die Erfindung erläutern, ohne ihre Anwendungsbreite einzuschränken, die sich aus der Beschreibung und den Patentansprüchen ergibt.

### Beispiel 1

### Herstellung eines Schwefeltrioxid/ DMF-Komplexes in DMF

In 3,3 kg über Molsiebe getrocknetes DMF wurde 10 h lang Kontaktofengas aus einer Schwefelsäureanlage mit 5,24 Vol-% Schwefeltrioxid mit einem Volumenstrom von 158 l/h eingeleitet. Die Reaktionstemperatur betrug dabei 10 °C. 98,1 % des Schwefeltrioxids wurden absorbiert, so dass eine Lösung mit 5,14 mol Schwefeltrioxid/DMF-Komplex entstand.

### Beispiel 2

### Durchführung der Sulfonierung

Zu der in Beispiel 1 hergestellten Lösung des Schwefeltrioxid/DMF-Komplexes wurde bei 8 °C 317 g (5,654 mol) flüssiges Isobuten während 0,5 h unter Rühren zudosiert. Dabei stellte sich ein Druck von 2,2- 2,3 bar ein. Das Reaktionsgemisch wurde in 0,5 h auf 45-46 °C erwärmt. Nach 0,2 h bei dieser Temperatur wurde der Ansatz auf 50 °C gebracht und 1,3 h bei 50 °C gerührt.

### Beispiel 3 (Vergleich)

### Neutralisierung der Reaktionslösung

Die aus Beispiel 2 enthaltene Reaktionslösung wurde neutralisiert, indem bei 50 °C 30 %ige Natronlauge in 0,5 h zugetropft wird. Die Ausbeute an Natriummethallylsulfonat im Reaktionsgemisch beträgt 95,2 % bezogen auf den Schwefeltrioxid/DMF-Komplex. 330 g DMF (10 %) gingen durch Hydrolyse verloren.

### Beispiel 4 (gemäß der Erfindung)

Das aus Beispiel 2 erhaltene Reaktionsgemisch wurde bei 50 °C mit 1,6 kg Wasser versetzt. Anschließend wurde bei 50 °C mit 30 %iger Natronlauge neutralisiert. Die Ausbeute an Natriummethallylsulfonat im Reaktionsgemisch betrug ebenfalls 95,2 % bezogen auf den Schwefeltrioxid/DMF-Komplex. Die Verluste an DMF durch Hydrolyse lagen unter 0,3 %.

Beispiel 4 zeigt im Vergleich mit Beispiel 3, dass im erfindungsgemäßen Verfahren durch Zugabe von Wasser vor der Neutralisation der DMF-Verlust durch Hydrolyse nahezu vollständig vermieden werden kann.

## Patentansprüche

1. Verfahren zur Herstellung von Salzen der Methallylsulfonsäure durch Umsetzung von Isobuten mit einem Schwefeltrioxid/Lewis-Base-Komplex in einem organischen Lösungsmittel, Neutralisation des entstehenden Reaktionsgemisches mit einer Base und Abtrennung des Methallylsulfonsäuresalz,
**dadurch gekennzeichnet,**
**dass**
a) Isobuten mit einem Schwefeltrioxid/Lewis-Base-Komplex bei - 20 bis 80 °C und 1 bis 15 bar sulfoniert,
b) das Reaktionsgemisch mit Wasser verdünnt,
c) das verdünnte Reaktionsgemisch mit einer Base auf einen pH von 6-8 neutralisiert und
d) das Methallylsulfonsäuresalz aus dem neutralisierten Reaktionsgemisch gewonnen wird, wobei das organische Lösungsmittel mit der als Komplexbildner eingesetzten Lewis-Base identisch ist.

2. Verfahren nach Anspruch 1,
**dadurch gekennzeichnet,**
**dass** als Lewis-Base N-N-dialkylierte Carbonsäureamide, N-alkylierte Lactame, cyclische Sulfoxide, cyclische Sulfone, Dialkylsulfone oder cyclische Ether eingesetzt werden.

3. Verfahren nach einem der Ansprüche 1 bis 2,
**dadurch gekennzeichnet,**
**dass** das Massenverhältnis von eingesetztem Schwefeltrioxid zu zugesetztem Wasser 1:2 bis 1:10 beträgt.

4. Verfahren nach einem der Ansprüche 1 bis 3,
**dadurch gekennzeichnet,**
**dass** Verfahrensschritt b) bei einer Temperatur von 20-80, insbesonders von 30 - 55 °C durchgeführt wird.

5. Verfahren nach Anspruch 1 bis 4,
**dadurch gekennzeichnet,**
**dass** das aus Verfahrensschritt c) erhaltene neutralisierte Reaktionsgemisch durch Destillation aufkonzentriert wird.

6. Verfahren nach Anspruch 1 bis 4,
**dadurch gekennzeichnet,**
**dass** das aus Verfahrensschritt c) erhaltene neutralisierte Reaktionsgemisch durch Elektrodialyse aufkonzentriert wird.

7. Verfahren nach einem der Ansprüche 5 oder 6,
**dadurch gekennzeichnet,**
**dass** die bei der Aufkonzentration anfallende Lewis-Base durch Destillation gereinigt und in den Prozess zurückgeführt wird.

8. Verfahren nach Anspruch 7,
**dadurch gekennzeichnet,**
**dass** die Lewis-Base nach Trocknung in den Prozess zurückgeführt wird.

9. Verfahren nach Anspruch 1 bis 8,
**dadurch gekennzeichnet,**
**dass** das Methallylsulfonsäuresalz aus dem ggf. aufkonzentrierten Reaktionsgemisch bei einer Temperatur von 20 - 30 °C auskristallisiert wird.

10. Verfahren nach einem der Ansprüche 1 bis 9,
**dadurch gekennzeichnet,**
**dass** das Reaktionsgemisch aus Verfahrensschritt a) durch Zugabe von Isobuten zum Schwefeltrioxid/Lewis-Base-Komplex bei -20 bis 25 °C hergestellt und anschließend auf bis zu 52 °C erwärmt wird.

11. Verfahren nach einem der Ansprüche 1 bis 10,
**dadurch gekennzeichnet,**
**dass** die Neutralisiation im Verfahrensschritt c) mit Carbonaten, Hydrogencarbonaten oder Hydroxiden der Alkali- oder Erdalkalimetalle, jeweils als Feststoff oder als wässrige Lösung, durchgeführt wird.

12. Verfahren nach einem der Ansprüche 1 bis 11,
**dadurch gekennzeichnet,**
**dass** die Sulfonierung mit einem 5 bis 60%igen molaren Überschuss an Isobuten bezüglich des Schwefeltroxid/Lewis-Base-Komplex durchgeführt wird.

## Claims

1. A process for preparing a salt of methallylsulphonic acid by reacting isobutene with a sulphur trioxide-Lewis base complex in an organic solvent, neutralizing the resulting reaction mixture by means of a base and separating off the salt of methallylsulphonic acid, **characterized in that**
a) isobutene is sulphonated by means of a sulphur trioxide-Lewis base complex at from -20 to 80°C and from 1 to 15 bar,
b) the reaction mixture is diluted with water,
c) the diluted reaction mixture is neutralized to a pH of 6-8 by means of a base, and
d) the salt of methallylsulphonic acid is isolated from the neutralized reaction mixture, the organic solvent being identical to the Lewis base used as complexing agent.

2. A process according to claim 1, **characterized in that** N,N-dialkylated carboxamide, N-alkylated lactam, cyclic sulphoxide, cyclic sulphone, dialkyl sulphone or cyclic ether is used as Lewis base.

3. A process according to either one of claims 1 and 2, **characterized in that** the mass ratio of sulphur trioxide used to water added is from 1:2 to 1:10.

4. A process according to any one of claims 1 to 3, **characterized in that** the process step b) is carried out at a temperature of 20-80°C, in particular 30-55°C.

5. A process according to any of claims 1 to 4, **characterized in that** the neutralized reaction mixture obtained from process step c) is concentrated by distillation.

6. A process according to any of claims 1 to 4, **characterized in that** the neutralized reaction mixture obtained from process step c) is concentrated by electrodialysis.

7. A process according to either one of claims 5 and 6, **characterized in that** the Lewis base obtained in the concentration procedure is purified by distillation and returned to the process.

8. A process according to claim 7, **characterized in that** the Lewis base is returned to the process after drying.

9. A process according to any of claims 1 to 8, **characterized in that** the salt of methallylsulphonic acid is crystallized from the reaction mixture, which may have been concentrated, at a temperature of 20-30°C.

10. A process according to any one of claims 1 to 9, **characterized in that** the reaction mixture of process step a) is prepared by addition of isobutene to the sulphur trioxide-Lewis base complex at from -20 to 25°C and subsequent heating to 52°C.

11. A process according to any one of claims 1 to 10, **characterized in that** the neutralization in process step c) is carried out using carbonate, hydrogencarbonate or hydroxide of an alkali metal or alkaline earth metal, in each case as a solid or as an aqueous solution.

12. A process according to any one of claims 1 to 11, **characterized in that** the sulphonation is carried out using a 5-60% molar excess of isobutene based on the sulphur trioxide-Lewis base complex.

## Revendications

1. Procédé pour la préparation de sels de l'acide méthallylsulfonique par réaction d'isobutène avec un complexe trioxyde de soufre/base de Lewis dans un solvant organique, neutralisation du mélange réactionnel résultant avec une base et séparation du sel de l'acide méthallylsulfonique,
**caractérisé en ce que**
a) on sulfone l'isobutène avec un complexe trioxyde de soufre/base de Lewis à - 20 à 80 °C et 1 à 15 bar,
b) on dilue le mélange réactionnel avec de l'eau,
c) on neutralise le mélange réactionnel dilué avec une base à un pH de 6 - 8, et
d) on obtient le sel de l'acide méthallylsulfonique à partir du mélange réactionnel neutralisé, le solvant organique étant identique à la base de Lewis utilisée comme agent complexant.

2. Procédé selon la revendication 1,
**caractérisé en ce que**
comme base de Lewis, on utilise des amides d'acides carboxyliques N-N-dialkylés, des lactames N-alkylés, des sulfoxydes cycliques, des sulfones cycliques, des dialkylsulfones ou des éthers cycliques.

3. Procédé selon l'une des revendications 1 ou 2,
**caractérisé en ce que**
le rapport massique du trioxyde de soufre utilisé à l'eau ajoutée est de 1/2 à 1/10.

4. Procédé selon l'une des revendications 1 à 3,
**caractérisé en ce que**
l'étape de procédé b) est effectuée à une température de 20 - 80, en particulier de 30 - 55 °C.

5. Procédé selon l'une des revendications 1 à 4,
**caractérisé en ce qu'**
on concentre par distillation le mélange réactionnel neutralisé obtenu dans l'étape c).

6. Procédé selon l'une des revendications 1 à 4,
**caractérisé en ce que**
on concentre par électrodialyse le mélange réactionnel neutralisé obtenu dans l'étape c).

7. Procédé selon l'une des revendications 5 ou 6,
**caractérisé en ce que**
la base de Lewis produite au cours de la concentration est purifiée par distillation et recyclée dans le processus.

8. Procédé selon la revendication 7,
**caractérisé en ce que**
la base de Lewis est recyclée dans le processus après séchage.

9. Procédé selon l'une des revendications 1 à 8,
**caractérisé en ce que**
le sel de l'acide méthallylsulfonique est séparé par cristallisation, à une température de 20 - 30 °C, du mélange réactionnel le cas échéant concentré.

10. Procédé selon l'une des revendications 1 à 9,
**caractérisé en ce que**
le mélange réactionnel obtenu dans l'étape a) est produit par addition d'isobutène au complexe trioxyde de soufre/base de Lewis à - 20 à 25 °C et est ensuite réchauffé jusqu'à 52 °C.

11. Procédé selon l'une des revendications 1 à 10,
**caractérisé en ce que**
la neutralisation dans l'étape c) est réalisée avec des carbonates, des hydrogénocarbonates ou des hydroxydes de métaux alcalins ou alcalino-terreux, à chaque fois sous forme de substance solide ou de solution aqueuse.

12. Procédé selon l'une des revendications 1 à 11,
**caractérisé en ce que**
la sulfonation est effectuée avec un excès molaire de 5 à 60 % d'isobutène par rapport au complexe trioxyde de soude/base de Lewis.
